(19) Europäisches Patentamt
European Patent Office
Office européen des brevets

(11) EP 0 755 674 B1

(12) FASCICULE DE BREVET EUROPEEN

(45) Date de publication et mention de la délivrance du brevet:
**15.12.1999 Bulletin 1999/50**

(51) Int. Cl.⁶: **A61K 7/48**

(21) Numéro de dépôt: **96401452.6**

(22) Date de dépôt: **01.07.1996**

(54) **Composition stable contenant de l'acide ascorbique**

Stabile Zusammensetzung, die Ascorbinsäure enthällt

Stable composition containing ascorbic acid

(84) Etats contractants désignés:
**DE ES FR GB IT**

(30) Priorité: **25.07.1995 FR 9509026**

(43) Date de publication de la demande:
**29.01.1997 Bulletin 1997/05**

(73) Titulaire: **L'OREAL**
**75008 Paris (FR)**

(72) Inventeurs:
- **Cantin, Hervé**
  **91420 Morangis (FR)**
- **Quemin, Eric**
  **93420 Villepinte (FR)**
- **Gagnebien, Didier**
  **92320 Chatillon (FR)**
- **Afriat, Isabelle**
  **75014 Paris (FR)**

(74) Mandataire: **Lhoste, Catherine**
**L'OREAL,**
**D.P.I.,**
**6, rue Bertrand Sincholle**
**92585 Clichy Cédex (FR)**

(56) Documents cités:
WO-A-90/12572          DE-A- 1 922 653
DE-A- 2 744 976          DE-B- 1 239 063
US-A- 4 983 382

- **DATABASE WPI Week 9205 Derwent Publications Ltd., London, GB; AN 92-035174 XP002002746 & JP-A-03 275 610 (KYOWA HAKKO KOGYO KK) , 6 Décembre 1991**
- **PATENT ABSTRACTS OF JAPAN vol. 4, no. 42 (C-5) [524] & JP-A-55 017313 (NIPPON SAAFUAKUTANTO KOGYO KK), 6 Février 1980,**

Remarques:
Le dossier contient des informations techniques présentées postérieurement au dépôt de la demande et ne figurant pas dans le présent fascicule.

## Description

La présente invention a pour objet une composition à application topique contenant de l'acide ascorbique stabilisé, utilisable en particulier dans les domaines cosmétique et/ou dermatologique.

L'invention se rapporte aussi à une utilisation de cette composition pour le traitement cosmétique de la peau ainsi que pour la fabrication d'une préparation destinée au traitement dermatologique de la peau.

L'invention se rapporte encore à un procédé de traitement cosmétique qui consiste à appliquer sur la peau ladite composition.

La composition de l'invention peut être appliquée, par voie topique, sur le visage, y compris autour des yeux, sur le corps ainsi que sur le cuir chevelu des êtres humains.

On cherche depuis longtemps à stabiliser l'acide ascorbique, ou vitamine C, dans des présentations galéniques appropriées, du fait de ses propriétés bénéfiques.

En effet, l'acide ascorbique possède de nombreuses fonctions biologiques comme la stimulation de la synthèse du collagène, le renfort des tissus cutanés contre les agressions extérieures (rayonnements UV, pollution), la dépigmentation, l'activité anti-radicaux libres, la compensation de la déficience en vitamine E. Certaines de ces propriétés bénéfiques ont été rapportées notamment par England et Seifter dans l'article "The biochemical functions of ascorbic acid" paru dans Ann. Rev. Nutri., 1986 : vol. 6, pp 365-406.

Cependant, en raison de sa structure chimique (d'alpha-cétolactone) l'acide ascorbique est très sensible à l'influence des paramètres de l'environnement comme la lumière, l'oxygène, l'eau (par son pH et par la présence de trace de métaux). Il s'ensuit une dégradation inéluctable au cours du temps de l'acide ascorbique en solution.

Dans l'état de la technique ce problème a été diversement traité.

Pour diminuer ou retarder la dégradation de l'acide ascorbique en solution, les auteurs du document US-A-5140043 ont préconisé de le stabiliser en l'introduisant dans des solutions hydroalcooliques, formées d'au moins 80 % d'eau et ayant un pH inférieur à 3,5.

En raison de la forte acidité de ces solutions, leur utilisation dans le domaine cosmétique et/ou pharmaceutique est difficilement envisageable.

En effet, une application répétée de ces solutions peut perturber l'équilibre de la peau et en particulier irriter, voire brûler la peau.

On connaît, par ailleurs, l'article de B.R. Hajratwala intitulé "Stability of ascorbic acid", paru dans la Revue Sciences Pharmaceutiques, le 15 Mars 1985. Le document US-A-4983383 divulgue une composition stable contenant de l'acide ascorbique, d'un polyol et de l'alcool.

Dans cet article il est enseigné notamment de stabiliser l'acide ascorbique en solution aqueuse acide par ajout d'un agent tensioactif qui est un ester de sorbitanne oxyéthyléné.

En outre, ce document enseigne l'emploi d'agent chélatant tel que l'éthylène diamine tétraacétique (EDTA) et le conditionnement sous azote, en l'absence de lumière, pour améliorer la stabilité de l'acide ascorbique en solution aqueuse.

Une telle solution aqueuse acide, appliquée sur la peau, présente les mêmes inconvénients que ceux décrits ci-dessus pour des solutions hydroalcooliques acides. De plus, la stabilisation obtenue est encore insuffisante.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agro-alimentaire).

Mais ces techniques sont d'une part coûteuses et peuvent d'autre part altérer l'acide ascorbique, par exemple lors d'un chauffage, et/ou conduire à des compositions peu cosmétiques, comme c'est le cas des granulés.

On connaît, par ailleurs, du document FR-A-1489249 l'emploi de sels métalliques d'acide ascorbique phosphorylé, notamment l'ascorbylphosphate de magnésium, dans des compositions cosmétiques.

Ce dernier composé a une activité proche de celle de l'acide ascorbique dont il est issu mais il présente certains inconvénients qui rendent son utilisation sur la peau peu probable. En particulier, l'ascorbylphosphate de magnésium n'étant stable qu'en pH basique (pH 8 à pH 9), il doit être incorporé dans une composition basique qui peut être irritante pour la peau (dont le pH a une valeur d'environ 5,5).

En conséquence, l'ensemble des propositions qui ont été faites jusqu'ici n'a pas permis de résoudre les problèmes techniques liés à l'instabilité de l'acide ascorbique en solution, dans une forme galénique appropriée pour les domaines cosmétique et/ou dermatologique et à un coût compatible avec les exigences industrielles.

Il subsiste donc le besoin d'une composition, utilisable dans les domaines cosmétique et/ou dermatologique, contenant de l'acide ascorbique stabilisé, à l'état libre, c'est à dire sans groupement additionnel notamment stabilisant, et qui ne provoque aucune irritation de la peau, après application.

La demanderesse a maintenant trouvé de manière inattendue que l'utilisation d'au moins un polyol liant l'eau dans une composition topique contenant l'acide ascorbique, en quantité efficace pour obtenir une valeur d'activité en eau de la composition, inférieure ou égale à 0,85, et d'au moins un agent structurant permet d'éviter la dégradation de l'acide ascorbique.

Aussi, la présente invention a pour objet une composition stable à application topique contenant de l'acide ascorbique et au moins un polyol, caractérisée en ce que la quantité de polyol seul est telle que l'on obtienue une valeur d'activité en eau de la composition, inférieure ou égale à 0,85 et en ce qu'elle comprend au moins un agent structurant choisi parmi les polymères et les huiles.

Certes, il est connu de préparer des compositions topiques à base d'acide ascorbique contenant des polyols, mais il n'a jamais été décrit ni suggéré que la seule présence de polyol et d'un agent structurant en une certaine quantité puisse éviter la dégradation de l'acide ascorbique. Ainsi, le document US-A-5140043 incite l'homme du métier à utiliser une concentration en glycol comprise entre 20 et 40 %, avec une quantité en eau au moins égale à celle des glycols. En revanche, il écarte l'homme du métier d'utiliser une concentration en glycol de 60 %.

Or, il a été maintenant trouvé que les polyols utilisés en quantité telle que la valeur d'activité en eau est au plus égale à 0,85 et en association avec un agent structurant, peuvent empêcher la dégradation de l'acide ascorbique.

Ainsi, la présente invention a aussi pour objet l'utilisation, dans une composition à application topique contenant de l'acide ascorbique, d'au moins un polyol en une quantité de polyol seul, efficace pour obtenir une valeur d'activité en eau de la composition, inférieure ou égale à 0,85, et d'au moins un agent structurant choisi parmi les polymères et les huiles, en vue de stabiliser l'acide ascorbique.

De préférence, le polymère est choisi parmi les polymères acryliques et méthacryliques.

De préférence, la quantité du ou des polyols doit être telle que la valeur d'activité en eau de la composition est inférieure ou égale à 0,7.

L'activité en eau $a_w$ d'un milieu contenant de l'eau est le rapport de la pression de vapeur d'eau du produit 〈〈 $P_{H2O}$ produit 〉〉 et de la pression de vapeur de l'eau pure 〈〈 $P_{H2O}$ pur 〉〉 à la même température. Elle peut être exprimée aussi comme le rapport du nombre de molécules d'eau 〈〈 $N_{H2O}$ 〉〉 sur le nombre de molécules totales 〈〈 $N_{H2O} + N_{corps\ dissous}$ 〉〉, qui tient compte de celles des corps dissous 〈〈 $N_{corps\ dissous}$ 〉〉.

Elle est donnée par les formules suivantes :

$$a_w = \frac{P_{H2O}\ produit}{P_{H2O}\ pur} = \frac{N_{H2O}}{N_{H2O} + N_{corps\ dissous}}$$

On peut utiliser différentes méthodes pour mesurer l'activité en eau. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable de l'activité en eau, ce qui signifie que la composition doit avoir une quantité minimum en eau, et en tout cas inférieure à celle du ou des polyols.

Le polyol utilisé selon l'invention peut être notamment choisi parmi la glycérine et les glycols, en particulier le propylène glycol et les polyéthylène glycols.

La quantité de polyol(s) à utiliser dans la composition de l'invention dépend du type de composition (gel ou émulsion) et des autres constituants présents dans la composition. Cette quantité doit être suffisante pour atteindre la valeur recherchée d'activité en eau. Le ou les polyols utilisés selon l'invention sont de préférence présents en une quantité d'au moins 30 % en poids, de préférence allant de 30 à 99,99 % en poids, et mieux de 45 à 80 % en poids par rapport au poids total de la composition.

On peut éventuellement remplacer une partie du polyol par un polymère acrylique ou méthacrylique contenant un ou des polyols et de l'eau complexés ou liés.

On entend par polymère acrylique ou méthacrylique un homopolymère ou un copolymère d'acide acrylique ou méthacrylique ou un homopolymère ou un copolymère d'un dérivé d'acide acrylique ou méthacrylique.

La quantité de tels polymères avec les polyols et l'eau liée, dans la composition selon l'invention va de préférence de 40 à 99,99 % en poids, et mieux de 60 à 90 % en poids par rapport au poids total de la composition.

On peut citer comme homopolymère de ce type ceux vendus sous les dénominations de Norgel et de Lubrajel CG par la société Guardian. Ces polymères sont des polyacrylates de glycéryle complexés avec plus de 65 % de glycérine et/ou de propylène glycol et moins de 35 % en poids d'eau liée.

Dans la composition selon l'invention, l'acide ascorbique peut être utilisé de manière avantageuse en une quantité allant de 0,05 à 10 % en poids, de préférence de 0,5 à 5 % en poids par rapport au poids total de la composition.

Comme huiles utilisables dans l'invention, on peut citer les huiles minérales (huile de vaseline), les huiles végétales (huile de jojoba), les huiles animales, les huiles de synthèse (oléate de décyle), les huiles siliconées (cyclométhicone, polydiméthylsiloxane, diméthicone) et les huiles fluorées (perfluoropolyéthers). La ou les huiles peuvent être présentes en une quantité allant de 5 à 60 %, et de préférence de 5 à 40 % en poids par rapport au poids total de la composition.

En outre, la composition selon l'invention peut contenir un ou plusieurs sels dont la présence va encore améliorer la stabilité de l'actif qu'elle contient. Comme sels, on peut citer en particulier les sels de magnésium et les sels de sodium, et plus spécialement le sulfate de magnésium, le chlorure de magnésium et le chlorure de sodium. Le ou les sels peuvent être présents en une quantité allant de 0,1 à 30 % et de préférence de 2 à 12 % en poids par rapport au poids total de la composition.

La composition selon l'invention contient un milieu topiquement acceptable, c'est-à-dire compatible avec la peau, et constitue notamment des compositions de protection, de traitement ou de soin de la peau.

Aussi, l'invention a encore pour objet l'utilisation de la composition ci-dessus pour le traitement cosmétique de la peau et en particulier pour lisser les ridules de la peau, la tonifier, la régénérer, pour éclaircir le teint, éliminer les taches pigmentaires de la peau, pour lutter contre les méfaits des rayonnements UV, et/ou pour renforcer de manière générale les tissus cutanés contre les agressions de l'environnement (pollution).

L'invention a également pour objet l'utilisation de la composition ci-dessus pour la fabrication d'une préparation destinée à un traitement dermatologique.

L'invention a enfin pour objet un procédé de traitement cosmétique qui consiste à appliquer sur la peau, y compris autour des yeux, une composition conforme à l'invention.

La composition selon l'invention peut se présenter notamment sous forme d'une solution, d'un gel, d'une émulsion eau-dans-huile ou huile-dans-eau constituant des crèmes, des onguents, des lotions ou des laits. Cette composition peut comprendre aussi des microcapsules, des microparticules, ou des dispersions vésiculaires de type ionique et/ou non ionique. Ces différentes formes de composition sont préparées selon les méthodes usuelles.

Lorsque la composition de l'invention est une émulsion, la proportion de la phase grasse peut aller de 10 à 80 % en poids, et de préférence de 20 à 40 % en poids par rapport au poids total de la composition. L'émulsion comprend de préférence au moins un agent dispersant choisi parmi les émulsionnants, les vésicules et les particules. Les huiles, les émulsionnants et éventuellement les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, par exemple en une proportion allant de 1 à 10 % en poids, et de préférence de 2 à 6 % en poids par rapport au poids total de la composition.

La phase grasse peut comprendre en plus des huiles indiquées ci-dessus des matières grasses telles que des alcools gras, des acides gras (acide stéarique), des cires (cire de silicone).

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles en plus de l'acide ascorbique, les gélifiants, les conservateurs, les antioxydants, les agents chélateurs, les solvants, les parfums, les charges, les filtres, les absorbeurs d'odeur et les matières colorantes. Les quantités de ces différents adjuvants sont celles classiquement utilisées dans les domaines considérés, et par exemple de 0,01 à 15 % du poids total de la composition. Ces adjuvants, selon leur nature, peuvent être introduits dans la phase grasse, dans la phase aqueuse et/ou dans les vésicules lipidiques.

Comme émulsionnants utilisables dans l'invention, on peut citer par exemple les émulsionnants siliconés comme les alkyldiméthicone copolyols tels que le cétyldiméthicone copolyol vendu par la société Goldschmidt sous la dénomination Abil EM-90, ou le mélange de diméthicone copolyol et cyclométhicone, vendu par la société Dow Corning sous la dénomination 3225C Formulation Aid.

Comme actifs hydrophiles, on peut utiliser les protéines ou les hydrolysats de protéine, les acides aminés, l'urée, l'allantoïne, les sucres et les dérivés de sucre, l'amidon, les extraits bactériens ou végétaux, notamment d'Aloe Vera.

Comme actifs lipophiles, on peut utiliser le tocophérol (vitamine E) et ses dérivés, le rétinol (vitamine A) et ses dérivés, les acides gras essentiels, les céramides, les huiles essentielles.

**Test de stabilité de l'activité de l'acide ascorbique :**

On a déterminé la stabilité de l'acide ascorbique à 2 % et à 5 % dans du Norgel après 2 mois à différentes températures.

| Gel | Acide ascorbique à T0 | Acide ascorbique à $T_{2\ mois}$ à 5°C | Acide ascorbique à $T_{2\ mois}$ à 20°C | Acide ascorbique à $T_{2\ mois}$ à 45°C |
|---|---|---|---|---|
| Gel à 2 % d'acide ascorbique | 2,05 ± 0,02 % | 2,09 ± 0,01 % | 2,09 ± 0,01 % | 1,94 ± 0,01 % |

(suite)

| Gel | Acide ascorbique à T0 | Acide ascorbique à $T_{2\,mois}$ à 5°C | Acide ascorbique à $T_{2\,mois}$ à 20°C | Acide ascorbique à $T_{2\,mois}$ à 45°C |
|---|---|---|---|---|
| Gel à 5 % d'acide ascorbique | 4,94 ± 0,03 % | 5,07 ± 0,01 % | 5,00 ± 0,05 % | 4,52 ± 0,04 % |

La stabilité de l'acide ascorbique après deux mois à 5°C ou à température ambiante est très bonne. Elle est encore satisfaisante après deux mois à 45°C.

Les exemples ci-après de compositions selon l'invention, sont donnés à titre d'illustration. Les quantités y sont données en % en poids.

**Exemple 1 : Gel**

| Norgel | 85 % |
|---|---|
| Acide ascorbique | 1 % |
| Eau | qsp 100 % |

On obtient un gel translucide, qui peut être appliqué chaque jour sur les taches du visage et du cou en vue de les diminuer. Son activité en eau est de 0,65 ± 0,02.

**Exemple 2 : Emulsion eau-dans-huile**

| _Phase huileuse :_ | |
|---|---|
| Cétyldiméthicone copolyol (Abil EM-90 vendu par la société Goldschmidt) (émulsionnant) | 2 % |
| Huile de jojoba | 4 % |
| Huile de vaseline | 10 % |
| Polydiméthylsiloxane | 8 % |
| _Phase aqueuse:_ | |
| Glycérine | 48,6 % |
| NaCl | 0,5 % |
| Acide ascorbique | 0,5 % |
| Eau | 26,4 % |

Le mode opératoire pour préparer l'émulsion est le suivant : On prépare la phase aqueuse d'une part et la phase huileuse d'autre part, et on émulsionne la phase aqueuse dans la phase huileuse à température ambiante sous agitation à l'homogénéisateur.

On obtient une crème blanche apte à faciliter le lissage des imperfections de la peau. Son activité en eau est de 0,63 ± 0,02.

**Exemple 3 : Emulsion eau-dans-huile**

| Phase huileuse : | |
|---|---|
| Diméthicone copolyol et cyclométhicone (《《3225C Formulation Aid 》》 vendu par Dow Corning) | 22,6 % |
| Diméthicone | 5 % |
| Huile minérale | 3 % |
| Phase aqueuse : | |
| Glycérine | 37 % |
| Sulfate de magnésium (stabilisant) | 2 % |
| Propylène glycol | 7 % |
| Acide ascorbique | 1 % |
| Eau | 22,4 % |

L'émulsion est préparée de la même manière que dans l'exemple 2.

On obtient une crème blanche dont l'application sur la peau procure un éclat du teint. Son activité en eau est de 0,63 ± 0,02.

**Exemple 4 : Emulsion eau-dans-huile**

| Phase huileuse : | |
|---|---|
| Diméthicone copolyol et cyclométhicone (《《 3225C Formulation Aid 》》 vendu par Dow Corning) | 8 % |
| Phényltriméthylsiloxytrisiloxane (《《 556 Fluid 》》 vendu par Dow Corning) | 15 % |
| Tocophérol | 0,5 % |
| Phase aqueuse : | |
| Propylène glycol | 39,4 % |
| Polyéthylène glycol 400 | 13 % |
| Sel disodique de l'acide éthylène diamine tétracétique (agent chélateur) | 0,1 % |
| Acide ascorbique | 3 % |
| Eau | 21 % |

L'émulsion est préparée de la même manière que dans l'exemple 2.

On obtient une crème blanche dont l'application sur la peau procure un éclat du teint. Son activité en eau est de 063 ± 0,02.

**Exemple 5 : Emulsion eau-dans-huile**

| Phase huileuse : | |
|---|---|
| Diméthicone copolyol et cyclométhicone (〈〈 3225C Formulation Aid 〉〉 vendu par Dow Corning) | 8 % |
| Phényltriméthylsiloxytrisiloxane (〈〈〈 556 Fluid 〉〉 vendu par Dow Corning) | 15 % |
| Tocophérol | 0,5 % |
| Phase aqueuse : | |
| Propylène glycol | 32,4 % |
| Polyéthylène glycol 400 | 13 % |
| Sel disodique de l'acide éthyléne diamine tétracétique (agent chélateur) | 0,1 % |
| Acide ascorbique | 3 % |
| Eau | 28 % |

L'émulsion est préparée de la même manière que dans l'exemple 2.

On obtient une crème blanche dont l'application sur la peau procure un éclat du teint. Son activité en eau est de 0,73 ± 0,02.

**Exemple comparatif 5 : Emulsion eau-dans-huile**

| Phase huileuse : | |
|---|---|
| Diméthicone copolyol et cyclométhicone (〈〈〈3225C Formulation Aid 〉〉 vendu par Dow Corning) | 8 % |
| Phényltriméthylsiloxytrisiloxane(〈〈 556 Fluid 〉〉 vendu par Dow Corning) | 15 % |
| Tocophérol | 0,5 % |
| Phase aqueuse : | |
| Sel disodique de l'acide éthylène diamine tétracétique (agent chélateur) | 0,1 % |
| Acide ascorbique | 3 % |
| Eau | 28 % |

L'émulsion est préparée de la même manière que dans l'exemple 2. Son activité en eau est de 0,95 ± 0,02.

On a déterminé la stabilité de l'acide ascorbique dans les compositions des exemples 4 et 5 et de l'exemple comparatif 5. Les résultats présentés dans le tableau suivant montre que les compositions des exemples 4 et 5 permettent un bon maintien de l'activité de l'acide ascorbique, ce qui n'est pas le cas de la composition de l'exemple comparatif 5 :

| Composition | % de dégradation de l'acide ascorbique à $T_2$ mois à 5°C | % de dégradation de l'acide ascorbique à $T_2$ mois à 20°C | % de dégradation de l'acide ascorbique à $T_2$ mois à 45°C |
|---|---|---|---|
| Exemple 4 ($a_w$ = 0,63) | 0 % | 0,7 % | 3,3 % |

(suite)

| Composition | % de dégradation de l'acide ascorbique à $T_2$ $_{mois}$ à 5°C | % de dégradation de l'acide ascorbique à $T_2$ $_{mois}$ à 20°C | % de dégradation de l'acide ascorbique à $T_2$ $_{mois}$ à 45°C |
|---|---|---|---|
| Exemple 5 ($a_w$ = 0,73) | 0 % | 3,5 % | 8 % |
| Exemple comparatif 5 ($a_w$ = 0,95) | 0 % | 6,2 % | 43 % |

Ces résultats montrent que la dégradation de l'acide ascorbique est d'autant plus importante que l'activité en eau de la composition est élevée.

## Revendications

1. Composition stable à application topique contenant de l'acide ascorbique et au moins un polyol, caractérisée en ce que la quantité de polyol seul est telle que l'on obtienne une valeur d'activité en eau de la composition, inférieure ou égale à 0,85, et en ce qu'elle comprend au moins un agent structurant choisi parmi les polymères et les huiles.

2. Composition selon la revendication 1, caractérisée en ce que le polyol est présent en une quantité efficace pour obtenir une valeur d'activité en eau de la composition, inférieure ou égale à 0,7.

3. Composition selon la revendication 1 ou 2, caractérisée en ce que le polyol est présent en une quantité allant de 30 à 99,99 % en poids par rapport au poids total de la composition.

4. Composition selon l'une quelconque des revendications précédentes, caractérisée en ce que le polyol est choisi dans le groupe comprenant la glycérine et les glycols.

5. Composition selon l'une quelconque des revendications précédentes, caractérisée en ce que le polymère est choisi parmi les polymères acryliques et méthacryliques.

6. Composition selon l'une quelconque des revendications précédentes, caractérisée en ce que le polymère comprend en outre de l'eau liée.

7. Composition selon l'une quelconque des revendications précédentes, caractérisée en ce que le polymère, le polyol et l'eau liée sont présents en une quantité allant de 70 à 99,99 % en poids par rapport au poids total de la composition.

8. Composition selon l'une quelconque des revendications précédentes, caractérisée en ce que l'huile est présente en une quantité allant de 5 à 60 % en poids par rapport au poids total de la composition.

9. Composition selon l'une quelconque des revendications précédentes, caractérisée en ce que l'huile est choisie parmi les huiles minérales, les huiles végétales, les huiles animales, les huiles de synthèse, les huiles siliconées et les huiles fluorées.

10. Composition selon l'une quelconque des revendications précédentes, caractérisée en ce qu'elle contient, en outre, au moins un sel inorganique.

11. Composition selon la revendication précédente, caractérisée en ce que le sel est choisi dans le groupe comprenant les sels de magnésium, les sels de calcium et les sels de sodium.

12. Composition selon la revendication 10 ou 11, caractérisée en ce que le ou les sels sont présents en une quantité allant de 0,1 à 30 % en poids par rapport au poids total de la composition.

13. Composition selon l'une quelconque des revendications précédentes, caractérisée en ce qu'elle se présente sous forme d'émulsion et en ce qu'elle comprend, en outre, au moins un agent dispersant choisi parmi les émulsionnants, les vésicules et les particules.

14. Composition selon l'une quelconque des revendications précédentes, caractérisée en ce que l'acide ascorbique est présent en une concentration allant de 0,05 à 10 % en poids par rapport au poids total de la composition.

15. Composition selon l'une quelconque des revendications précédentes, caractérisée en ce que la composition comprend au moins un adjuvant lipophile ou hydrophile choisi parmi les conservateurs, les antioxydants, les agents chélateurs, les parfums, les charges, les filtres, les séquestrants, les huiles essentielles, les matières colorantes, les actifs hydrophiles ou lipophiles, et les vésicules lipidiques.

16. Composition stable à application topique contenant de l'acide ascorbique et au moins un polyol, caractérisée en ce que le polyol est présent en une quantité efficace pour obtenir une valeur d'activité en eau de la composition, inférieure ou égale à 0,85, et en ce qu'elle comprend au moins un agent structurant choisi parmi les polymères et les huiles et au moins un émulsionnant siliconé et/ou un sel.

17. Utilisation, dans une composition à application topique contenant de l'acide ascorbique, d'au moins un polyol en une quantité de polyol seul, efficace pour obtenir une valeur d'activité en eau de la composition, inférieure ou égale à 0,85, et d'au moins un agent structurant choisi parmi les polymères et les huiles, en vue de stabiliser l'acide ascorbique.

18. Utilisation selon la revendication précédente, caractérisée en ce que le polymère est choisi parmi les polymères acryliques et méthacryliques.

19. Utilisation de la composition selon l'une quelconque des revendications 1 à 15 pour le traitement cosmétique de la peau et en particulier pour lisser les ridules de la peau, la tonifier, la régénérer, pour éclaircir le teint, éliminer les taches pigmentaires de la peau, pour lutter contre les méfaits des rayonnements UV, et/ou pour renforcer de manière générale les tissus cutanés contre les agressions de l'environnement.

20. Utilisation de la composition selon l'une quelconque des revendications 1 à 15 pour la fabrication d'une préparation destinée à un traitement dermatologique.

21. Procédé de traitement cosmétique qui consiste à appliquer sur la peau, y compris autour des yeux, une composition selon l'une quelconque des revendications 1 à 15.

## Claims

1. Stable composition for topical application, containing ascorbic acid and at least one polyol, characterized in that the amount of polyol alone is such that a water activity value for the composition is obtained which is less than or equal to 0.85, and in that it comprises at least one structuring agent chosen from polymers and oils.

2. Composition according to Claim 1, characterized in that the polyol is present in an amount that is effective for obtaining a water activity value for the composition which is less than or equal to 0.7.

3. Composition according to Claim 1 or 2, characterized in that the polyol is present in an amount ranging from 30 to 99.99 % by weight, relative to the total weight of the composition.

4. Composition according to any one of the preceding claims, characterized in that the polyol is chosen from the group comprising glycerol and glycols.

5. Composition according to any one of the preceding claims, characterized in that the polymer is chosen from acrylic and methacrylic polymers.

6. Composition according to any one of the preceding claims, characterized in that the polymer also comprises bound water.

7. Composition according to any one of the preceding claims, characterized in that the polymer, the polyol and the bound water are present in an amount ranging from 70 to 99.99% by weight relative to the total weight of the composition.

8. Composition according to any one of the preceding claims, characterized in that the oil is present in an amount ranging from 5 to 60% by weight relative to the total weight of the composition.

9. Composition according to any one of the preceding claims, characterized in that the oil is chosen from mineral oils, plant oils, animal oils, synthetic oils, silicone oils and fluoro oils.

10. Composition according to any one of the preceding claims, characterized in that it also contains at least one inorganic salt.

11. Composition according to the preceding claim, characterized in that the salt is chosen from the group comprising magnesium salts, calcium salts and sodium salts.

12. Composition according to Claim 10 or 11 characterized in that the salt(s) is(are) present in an amount ranging from 0.1 to 30% by weight relative to the total weight of the composition.

13. Composition according to any one of the preceding claims, characterized in that it is in the form of an emulsion and in that it also comprises at least one dispersing agent chosen from emulsifiers, vesicles, and particles.

14. Composition according to any one of the preceding claims, characterized in that ascorbic acid is present in a concentration ranging from 0.05 to 10 % by weight, relative to the total weight of the composition.

15. Composition according to any one of the preceding claims, characterized in that the composition includes at least one lipophilic or hydrophilic adjuvant chosen from preserving agents, antioxidants, chelating agents, perfumes, fillers, screening agents, sequestering agents, essential oils, dyes, hydrophilic or lipophilic active substances and lipid vesicles.

16. Stable composition for topical application containing ascorbic acid and at least one polyol, characterized in that the polyol is present in an amount which is effective for obtaining a water activity value for the composition of less than or equal to 0.85, and in that it comprises at least one structuring agent chosen from polymers and oils and at least one silicone emulsifier and/or a salt.

17. Use, in a composition for topical application, containing ascorbic acid, of at least one polyol in an amount of polyol alone which is effective for obtaining a water activity value for the composition which is less than or equal to 0.85, and of at least one structuring agent chosen from polymers and oils, with a view to stabilizing the ascorbic acid.

18. Use according to the preceding claim characterized in that the polymer is chosen from acrylic and methacrylic polymers

19. Use of the composition according to any one of Claims 1 to 15 for the cosmetic treatment of the skin and in particular for smoothing out fine lines in the skin, toning it up, regenerating it, for lightening the complexion, removing pigmentary marks from the skin, for combating the detrimental effects of UV radiation, and/or for generally reinforcing skin tissues against attack by the environment.

20. Use of the composition according to any one of Claims 1 to 15 for the manufacture of a preparation intended for a dermatological treatment.

21. Process of cosmetic treatment which consists in applying to the skin, including the area around the eyes, a composition according to any one of Claims 1 to 15.

**Patentansprüche**

1. Stabile Zusammensetzung zur topischen Anwendung, die Ascorbinsäure und mindestens ein Polyol enthält, dadurch gekennzeichnet, daß die Menge des Polyols allein so ist, daß ein Wert der Wasseraktivität der Zusammensetzung von höchstens 0,85 erzielt wird, und dadurch, daß sie mindestens ein Strukturierungsmittel enthält, das unter Polymeren und Ölen ausgewählt ist.

2. Zusammensetzung nach Anspruch 1, dadurch gekennzeichnet, daß das Polyol in einer Menge vorliegt, die so wirksam ist, daß ein Wert der Wasseraktivität der Zusammensetzung von höchstens 0,7 erzielt wird.

3. Zusammensetzung nach Anspruch 1 oder 2, dadurch gekennzeichnet, daß das Polyol in einem Mengenanteil von 30 bis 99,99 Gew.-%, bezogen auf das Gesamtgewicht der Zusammensetzung, vorliegt.

4. Zusammensetzung nach einem der vorhergehenden Ansprüche, dadurch gekennzeichnet, daß das Polyol unter Glycerin und Glykolen ausgewählt ist.

5. Zusammensetzung nach einem der vorhergehenden Ansprüche, dadurch gekennzeichnet, daß das Polymer unter Acrylpolymeren und Methacrylpolymeren ausgewählt ist.

6. Zusammensetzung nach einem der vorhergehenden Ansprüche, dadurch gekennzeichnet, daß das Polymer ferner gebundenes Wasser enthält.

7. Zusammensetzung nach einem der vorhergehenden Ansprüche, dadurch gekennzeichnet, daß das Polymer, das Polyol und das gebundene Wasser in einem Mengenanteil von 70 bis 99,99 Gew.-%, bezogen auf das Gesamtgewicht der Zusammensetzung, vorliegen.

8. Zusammensetzung nach einem der vorhergehenden Ansprüche, dadurch gekennzeichnet, daß das Öl in einem Mengenanteil von 5 bis 60 Gew.-%, bezogen auf das Gesamtgewicht der Zusammensetzung, vorliegt.

9. Zusammensetzung nach einem der vorhergehenden Ansprüche, dadurch gekennzeichnet, daß das Öl ausgewählt ist unter Mineralölen, pflanzlichen Ölen, tierischen Ölen, synthetischen Ölen, Siliconölen und fluorierten Ölen.

10. Zusammensetzung nach einem der vorhergehenden Ansprüche, dadurch gekennzeichnet, daß sie ferner mindestens ein anorganisches Salz enthält.

11. Zusammensetzung nach dem vorhergehenden Anspruch, dadurch gekennzeichnet, daß das Salz ausgewählt ist unter Magnesiumsalzen, Calciumsalzen und Natriumsalzen.

12. Zusammensetzung nach Anspruch 10 oder 11, dadurch gekennzeichnet, daß das Salz oder die Salze in einem Mengenanteil von 0,1 bis 30 Gew.-%, bezogen auf das Gesamtgewicht der Zusammensetzung, vorliegen.

13. Zusammensetzung nach einem der vorhergehenden Ansprüche, dadurch gekennzeichnet, daß sie in Form einer Emulsion vorliegt und dadurch, daß sie ferner mindestens ein Dispergiermittel enthält, das unter Emulgatoren, Vesikeln und Partikeln ausgewählt ist.

14. Zusammensetzung nach einem der vorhergehenden Ansprüche, dadurch gekennzeichnet, daß die Ascorbinsäure in einer Konzentration von 0,05 bis 10 Gew.-%, bezogen auf das Gesamtgewicht der Zusammensetzung, vorliegt.

15. Zusammensetzung nach einem der vorhergehenden Ansprüche, dadurch gekennzeichnet, daß die Zusammensetzung mindestens einen lipophilen oder hydrophilen Zusatzstoff enthält, der ausgewählt ist unter Konservierungsmitteln, Antioxidantien, Chelatbildnern, Parfums, Füllstoffen, Filtern, Maskierungsmitteln, etherischen Ölen, Färbemitteln, hydrophilen oder lipophilen Wirkstoffen und Lipidvesikeln.

16. Stabile Zusammensetzung zur topischen Anwendung, die Ascorbinsäure und mindestens ein Polyol enthält, dadurch gekennzeichnet, daß das Polyol in einer Menge vorliegt, die so wirksam ist, daß ein Wert der Wasseraktivität der Zusammensetzung von höchstens 0,85 erzielt wird, und dadurch, daß sie mindestens ein Strukturierungsmittel, das unter Polymeren und Ölen ausgewählt ist, und mindestens einen Siliconemulgator und/oder ein Salz enthält.

17. Verwendung mindestens eines Polyols und mindestens eines Strukturierungsmittels in einer Zusammensetzung zur topischen Anwendung, die Ascorbinsäure enthält, wobei das Polyol in einer Menge verwendet wird, die allein so wirksam ist, daß ein Wert der Wasseraktivität der Zusammensetzung von höchstens 0,85 erzielt wird, und wobei das Strukturierungsmittel unter Polymeren und Ölen ausgewählt ist, um die Ascorbinsäure zu stabilisieren.

18. Verwendung nach dem vorhergehenden Anspruch, dadurch gekennzeichnet, daß das Polymer unter Acrylpolymeren und Methacrylpolymeren ausgewählt ist.

19. Verwendung der Zusammensetzung nach einem der Ansprüche 1 bis 15 zur kosmetischen Behandlung der Haut und insbesondere, um Hautfältchen zu glätten, die Haut zu beleben und zu regenerieren, den Teint aufzufrischen, Pigmentflecken der Haut zu entfernen, negative Folgen der UV-Strahlung zu bekämpfen und/oder allgemein das Hautgewebe gegen Umwelteinwirkungen zu stärken.

20. Verwendung der Zusammensetzung nach einem der Ansprüche 1 bis 15 zur Herstellung eines Präparats, das für eine dermatologische Behandlung bestimmt ist.

21. Verfahren zur kosmetischen Behandlung, das darin besteht, eine Zusammensetzung nach einem der Ansprüche 1 bis 15 auf die Haut einschließlich der Augenpartie aufzutragen.